(19) Europäisches Patentamt
European Patent Office
Office européen des brevets

(11) Veröffentlichungsnummer: **0 176 076**
**B1**

(12) # EUROPÄISCHE PATENTSCHRIFT

(45) Veröffentlichungstag der Patentschrift: **14.09.88**

(51) Int. Cl.$^4$: **C 07 D 323/00**

(21) Anmeldenummer: **85112084.0**

(22) Anmeldetag: **24.09.85**

(54) **Verfahren zur Herstellung von Kronenethern.**

(30) Priorität: **24.09.84 DE 3435005**

(43) Veröffentlichungstag der Anmeldung: **02.04.86 Patentblatt 86/14**

(45) Bekanntmachung des Hinweises auf die Patenterteilung: **14.09.88 Patentblatt 88/37**

(84) Benannte Vertragsstaaten: **BE DE FR GB IT NL**

(56) Entgegenhaltungen:
**DD - A - 212 963**
**DE - A - 2 153 844**
**DE - A - 2 201 583**
**DE - A - 2 539 324**
**DE - B - 1 518 103**
**US - A - 4 421 923**

**CHEMICAL ABSTRACTS, vol. 92, no. 11, March 17, 1980, Columbus, Ohio, USA; OKAHARA, MITSUO; IKEDA, ISAO; YANAGIDA, SHOZO (MOBAY CHEMICAL CORP.) "Crown ethers", page 599, column 1,abstract no. 94451x**

(73) Patentinhaber: **Consortium für elektrochemische Industrie GmbH, Zielstattstrasse 20, D-8000 München 70 (DE)**

(72) Erfinder: **Gamon, Nobert, Dr. Dipl.-Chem., Mühlbachstrasse 52, D-8261 Emmerting (DE)**
Erfinder: **Gebhart, Christine, Ebermayerstrasse 20, D-8000 München 70 (DE)**
Erfinder: **Schawohl, Georg, Kafkastrasse 18, D-8000 München 83 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft ein Verfahren zur Herstellung von makrocyclischen Polyethern durch Cyclisieren von α,ω-Diolen mit α,ω-Dichloriden oder durch Cyclisieren von α,ω-Chlorhydrinen in Gegenwart von Kaliumhydroxid und Dimethylsulfoxid.

Derartige, auch als Kronenether bezeichnete makrocyclische Verbindungen werden u.a. als Phasentransfer-Katalysatoren eingesetzt. Die genannten Verbindungen waren bisher schwer zugänglich, so dass ihre Anwendung, insbesondere aus Kostengründen, auf den Labormassstab beschränkt war.

Es ist gemäss Topics in Curr. Chem. 113, S. 29, bekannt, Kronenether durch Cyclisieren von α,ω-Diolen mit α,ω-Dichloriden in Gegenwart von beispielsweise Kaliumhydroxid und Dimethylsulfoxid als Lösungsmittel herzustellen. Es werden jedoch, bei wenig selektivem Reaktionsverlauf bezüglich der eingesetzten Chlorkomponente, relativ bescheidene Ausbeuten erzielt.

Aufgabe der Erfindung war es, einen verbesserten Syntheseweg für Kronenether aufzufinden.

Es wurde nun gefunden, dass die Synthese von Kronenethern in Gegenwart von Dimethylsulfoxid und Kaliumhydroxid mit verbesserten Ausbeuten verläuft, wenn das Reaktionsgemisch grössere Mengen an Wasser enthält.

Gegenstand der Erfindung ist ein Verfahren zur Herstellung von makrocyclischen Kronenethern, die aus gleichen oder verschiedenen Struktureinheiten der Formel

$$-[CR^1R^2-CR^3R^4-A]_x-$$

aufgebaut sind, wobei

A für etherischen Sauerstoff und die Gruppe

$$\begin{array}{c} | \\ -N-R^5 \end{array}$$

steht,

$R^1$, $R^2$, $R^3$, $R^4$ und $R^5$ Wasserstoff, Alkyl mit 1 bis 18 Kohlenstoffatomen, Alkoxymethyl mit 1 bis 18 Kohlenstoffatomen im Alkoxyrest, Alkenyloxymethyl mit 2 bis 18 Kohlenstoffatomen im Alkenyloxyrest, Alkoxyethyl mit 1 bis 18 Kohlenstoffatomen im Alkoxyrest, Alkenyloxyethyl mit 2 bis 18 Kohlenstoffatomen im Alkenyloxyrest, Alkoxypolyethoxymethyl mit 1 bis 3 Ethylenoxy-Einheiten, Alkoxypolyethoxyethyl mit 1 bis 3 Ethylenoxy-Einheiten, Alkenyl mit 3 bis 18 Kohlenstoffatomen, Phenyl, Phenyloxymethyl, Benzyl und Benzyloxymethyl bedeuten und

x eine ganze Zahl von 5 bis 10 ist, durch

a) Cyclisieren von entsprechende Struktureinheiten aufweisenden α,ω-Diolen mit entsprechende Struktureinheiten aufweisenden α,ω-Dichloriden oder

b) durch Cyclisieren von entsprechende Struktureinheiten aufweisenden α,ω-Chlorhydrinen

in Gegenwart von Kaliumhydroxid unter Verwendung von Dimethylsulfoxid als Lösungsmittel, das dadurch gekennzeichnet ist, dass die Cyclisierungsreaktion in Gegenwart von 18 bis 50 Gew.-%, bezogen auf das Gesamtgewicht des Gemisches Wasser/Dimethylsulfoxid/Kaliumhydroxid, an Wasser durchgeführt wird, das zusätzlich zu ggf. bei der Cyclisierungsreaktion gebildetem Wasser dem Reaktionsgemisch zugeführt wurde.

Vorzugsweise stehen $R^1$, $R^2$, $R^3$, $R^4$ und $R^5$ für Wasserstoff, Methyl, Ethyl, n-Propyl, n-Butyl, Methoxymethyl, Ethoxymethyl, Propoxymethyl, Allyloxymethyl, Crotyloxymethyl, 2-Methoxyethyl, 2-Ethyloxyethyl, 2-n-Propyloxyethyl, 2-n-Butyloxyethyl, Allyl, Crotyl, Prenyl, Phenyl, Phenyloxymethyl, 2-Phenyloxyethyl, Benzyl, Benzyloxymethyl, 2-Benzyloxyethyl, (2-Ethoxy-ethoxy)-methyl, 2-(2-Ethoxy-ethoxy)-ethyl, (2-Methoxyethoxy)-methyl, [2.(2-Methoxyethoxy)-ethoxy]-methyl, [2-(2-Ethoxyethoxy)-ethoxy]-methyl, 2-[2-(2-Methoxyethoxy)-ethoxy]-ethyl, 2-[2-(2-Ethoxyethoxy)- ethoxy]-ethyl, n-Butoxymethyl, tert.-Butoxymethyl, 2-tert.-Butoxyethyl und andere.

Für $R^1$, $R^2$, $R^3$ und $R^4$ sind unter den Alkoxyalkylgruppen (einschliesslich der Alkenyloxyalkyl-Gruppen und der Alkoxy-polyethoxyalkyl-Gruppen) die Alkoxymethyl-Gruppen und für $R^5$ die Alkoxyethyl-Gruppen bevorzugt.

Unter den Alkoxy-polyethoxyalkyl-Gruppen sind solche Gruppen bevorzugt, bei denen Alkoxy für Methoxy und Ethoxy steht.

x ist vorzugsweise 6.

Beispiele für erfindungsgemäss herzustellende Verbindungen sind 18-Krone-6, Methyl-18-Krone-6, Ethyl-18-Krone-6, Propyl-18-Krone-6, Butyl-18-Krone-6, Dimethyl-18-Krone-6,

Trimethyl-18-Krone-6, Methoxymethyl-18-Krone-6, Ethoxymethyl-18-Krone-6, tert.-Butoxymethyl-18-Krone-6, Allyloxymethyl-18-Krone-6, Phenoxymethyl-18-Krone-6, Benzyloxymethyl-18-Krone-6, (2-Methoxyethoxy)methyl-18-Krone-6, (2-Ethoxyethoxy)methyl-18-Krone-6, N-Methyl-1-aza-18-Krone-6, N-Ethyl-1-aza-18-Krone-6, N-Allyl-1-aza-18-Krone-6, N-Benzyl-1-aza-18-Krone-6 und andere.

Die als Ausgangsverbindungen einzusetzenden α,ω-Diole lassen sich durch die allgemeine Formel

$$OH-[CR^1R^2-CR^3R^4-A]_m-CR^1R^2-CR^3R^4-OH$$

darstellen, wobei $R^1$, $R^2$, $R^3$, $R^4$ und A die oben gegebene Bedeutung besitzen und

m eine ganze Zahl von 0 bis 7 ist.

Spezielle Beispiele für erfindungsgemäss einzusetzende α,ω-Diole sind Ethylenglycol, Diethylenglycol, Triethylenglycol, Tetraethylenglycol, Pentaethylenglycol, Hexaethylenglycol, Heptaethylenglycol, Octaethylenglycol, Propylenglycol, Dipropylenglycol, Tripropylenglycol, 1,2-Butylenglycol, Methyldiethylenglycol, Methyltriethylenglycol, Methyltetraethylenglycol, Methoxymethyl-ethylenglycol, Methoxymethyl-diethylenglycol, Methoxymethyl-triethylenglycol, Methoxymethyl-tetraethylenglycol, Ethoxymethylethylenglycol, Ethoxymethyldiethylenglycol, Ethoxymethyl-triethylenglycol, Ethoxymethyl-tetraethylenglycol, n-Butoxymethylethylenglycol, n-Butoxymethyl-diethylenglycol, n-Butoxymethyltriethylenglycol, Butoxymethyl-tetraethylenglycol, tert.-Butoxymethyl-ethylenglycol, tert.-Butoxymethyl-diethylenglycol, tert.-Butoxymethyl-triethylenglycol, tert.-Butoxymethyl-tetraethylenglycol, Allyloximethyl-ethylenglycol, Allyloximethyl-diethylenglycol, Allyloxymethyl-triethylenglycol, Allyloxymethyl-tetraethylenglycol, Phenoxymethylethylenglycol, Phenoxymethyl-diethylenglycol, Phenoxymethyl-triethylenglycol, Phenoxymethyl-tetraethylenglycol, Benzyloxymethyl-ethylenglycol, Benzyloxymethyl-diethylenglycol, Benzyloxymethyl-triethylenglycol, Benzyloxymethyl-tetraethylenglycol, N-Methyl-diethanolamin, N-Ethyl-diethanolamin, N-Allyl-diethanolamin, N-Benzyl-diethanolamin, N-(2-Methoxyethyl)-diethanolamin und andere.

Die erfindungsgemäss als Ausgangsverbindungen einzusetzenden α,ω-Dichloride sind durch die Formel

$$Cl-[CR^1R^2-CR^3R^4-A]_n-CR^1R^2-CR^3R^4-Cl$$

darstellbar.

n ist eine ganze Zahl von 1 bis 7.

Spezielle Beispiele für α,ω-Dichloride sind 2,2'-Dichlordiethylether, 1,2-Bis(2-Chlorethoxy) ethan, Bis[2-(2-Chlorethoxy)ethyl]-ether, β,β'-Dichlordipropylether und andere.

Die Auswahl der einzusetzenden α,ω-Diole und α,ω-Dichloride für die Cyclisierungsreaktion erfolgt nach Massgabe der erwünschten Ringgrösse des Kronenethers und seiner Substituenten.

Alternativ können als Ausgangssubstanzen zu der Kombination α,ω-Diol/α,ω-Dichlorid auch α,ω-Chlorhydrine eingesetzt werden. Die erfindungsgemäss einzusetzenden α,ω-Chlorhydrine lassen sich durch die allgemeine Formel

$$OH-[CR^1R^2-CR^3R^4-A]_p-CR^1R^2-CR^3R^4-Cl$$

darstellen.

p ist eine ganze Zahl von 2 bis 9.

Spezielle Beispiele sind 1-(2-Chlorethoxy)-2-(2-hydroxyethoxy)- ethan, 2-(2-Chlorethoxy) ethyl-2-(2-hydroxyethoxy)ethylether, 2-[2-(2-Chlorethoxy)ethoxy]ethyl- 2-[2-(2-hydroxyethoxy)ethoxy]ethylether und andere.

Kaliumhydroxid kommt in fester Form oder in Lösung, insbesondere in wässriger Lösung, zum Einsatz. Die Menge an Kaliumhydroxid wird abgestimmt auf die an sich bei der Cyclisierungsreaktion frei werdende Menge an HCl. Es werden pro Mol HCl 0,9 bis 1,5 Mol KOH, insbesondere 1,0 bis 1,4 Mol KOH, eingesetzt.

Die Menge an Dimethylsulfoxid wird abgestimmt auf die Menge der umzusetzenden, an Kohlenstoff gebundenen OH-Gruppen. Sie beträgt pro 1 Mol an Kohlenstoff gebundener OH-Gruppen 0,7 bis 7 Mol, insbesondere 1,5 bis 5 Mol Dimethylsulfoxid.

Die Menge an erfindungsgemäss einzusetzendem Wasser, das zusätzlich zu ggf. bei der Cyclisierungsreaktion entstehendem Wasser eingesetzt wird, wird abgestimmt auf die Gesamtmenge des Gemisches KOH/Dimethylsulfoxid/Wasser und beträgt 18 bis 50 Gew.-%, insbesondere 20 bis 35 Gew.-%.

Sofern α,ω-Diole mit α,ω-Dichloriden zur Reaktion gebracht werden, beträgt die Menge der einzusetzenden Dichlorkomponente 1 bis 2 Mol, bezogen auf 1 Mol Diolkomponente.

Zur Durchführung des erfindungsgemässen Verfahrens wird ein Reaktionsgemisch aus Kaliumhydroxid, Dimethylsulfoxid, Wasser und α,ω-Diol sowie α,ω-Dichlorid bzw. α,ω-Chlorhydrin bei Temperaturen von 0 bis 40 °C gerührt. Falls erwünscht, kann das Reaktionsgemisch vor Beendigung der Reaktion (z.B. wenn etwa 80% des zu erzielenden Umsatzes erreicht sind) noch auf Temperaturen von 60 bis 100 °C erwärmt werden.

Die Aufarbeitung des Reaktionsgemisches kann nach an sich bekannten Methoden erfolgen: in der Regel werden zunächst die anorganischen Bestandteile abgetrennt, die Lösungsmittel abgezogen und das Zielprodukt durch Destillation, Chromatographie, Kristallisation, Komplexbildung oder Extraktion isoliert. Es hat sich insbesondere bewährt, eine Extraktion in Gegenwart von Ethylenglycol vorzunehmen.

Nach dem erfindungsgemässen Verfahren gelingt es, Kronenether unter moderaten Reaktionsbedingungen in hoher Ausbeute und Reinheit herzustellen.

Die Erfindung wird nun anhand von Beispielen näher erläutert:

Beispiel 1

Herstellung von 18-Krone-6

138 kg Tetraethylenglycol, 152 kg 2,2'-Dichlordiethylether, 257 kg einer 50 Gew.-%igen Lösung von KOH in Wasser und 306 kg Dimethylsulfoxid wurden bei einer Temperatur von 15 °C 68 Stunden gerührt. Danach wurde das Gemisch innerhalb von 8 Stunden auf 100 °C erhitzt und noch 1 Stunde unter Rühren bei dieser Temperatur belassen. Danach wurde abgekühlt und mit Salzsäure angesäuert. Anschliessend wurde evakuiert, um in einem Temperaturintervall von 25 bis 30 °C den als Nebenprodukt entstandenen Acetaldehyd abzudestillieren. Danach wurde mit Kaliumhydroxid neutralisiert und der entstandene Salzkuchen abfiltriert. Vom Filtrat wurden anschliessend Wasser und Dimethylsulfoxid abdestilliert und der Rückstand in 500 l Toluol aufgenommen. Es wurde auf eine Temperatur von 120 °C erwärmt und nochmals filtriert. Die toluolische Lösung wurde eingeengt, anschliessend mit 150 kg Ethylenglycol versetzt und in einer Pulsations-Füllkörperkolonne (Durchmesser 80 mm, Länge 6 m) bei 80 °C im Gegenstrom mit 1000 l Cyclohexan extrahiert. Hierzu wurde das Gemisch der Kolonne im oberen Drittel aufgegeben, während über Kopf der Kolonne noch 100 kg Ethylenglycol zugegeben wurden. Schliesslich wurde vom Extrakt Cyclohexan abdestilliert und das im Extrakt noch vorhandene Ethylenglycol durch azeotrope Destillation mit Toluol entfernt. Nach

dem Einengen verblieben 116.kg an Zielprodukt, das zunächst als leicht gelbliche Flüssigkeit anfiel, die langsam erstarrte. Die Ausbeute, bezogen auf eingesetztes Tetraethylenglycol betrug 62%.

Schmelzpunkt 38 bis 40 °C, Reinheit 98,4%.

Beispiel 2

[2-(2-Ethoxyethoxy)ethoxy]methyl-18-Krone-6

a) Zu einer Mischung aus 278 g Diethylenglycolmonoethylether, 0,5 ml Bortrifluorid-etherat und 50 ml Dichlormethan wurden bei 45 °C 46,3 g Epichlorhydrin innerhalb von 30 Minuten zugetropft. Anschliessend wurde 2 Stunden unter Rückfluss gekocht, überschüssiges Ausgangsmaterial abdestilliert und der Rückstand fraktioniert destilliert. Es wurden 98 g, entsprechend 80% d.Th. an 1-Chlor-3- [2-(2-Ethoxyethoxy)ethoxy]propan-2-ol erhalten.

b) 21,8 g Kaliumhydroxid wurden 100 °C in 225 g Triethylenglycol gelöst. Nach dem Abkühlen auf 10 °C wurden 67,9 g 1-Chlor-3-[2-(2-ethoxyethoxy)ethoxy]propan-2-ol zugetropft. Es wurde anschliessend noch 1 Stunde bei Raumtemperatur gerührt und danach auf 120 °C erwärmt. Schliesslich wurde die Hauptmenge an überschüssigem Triethylenglycol im Vakuum abdestilliert, der Rückstand mit 150 ml Dichlormethan versetzt und anschliessend filtriert. Nach Abziehen des Dichlormethans wurde fraktioniert destilliert. Es fielen 66 g an [2-(2-Ethoxyethoxy) ethoxy]methyl-tetraethylenglycol als ölige Flüssigkeit an, entsprechend einer Ausbeute von 64%.

c) 64,6 g [2-(2-Ethoxyethoxy)ethoxy]methyltetraethylenglycol, 40,7 g 2,2'-Dichloridethylether, 66,2 g einer 51 Gew.-%igen wässrigen Lösung von KOH und 100 ml Dimethylsulfoxid wurden 72 Stunden bei 15 °C gerührt. Danach wurde die Mischung auf 100 °C innerhalb von 4 Stunden aufgeheizt und noch 2 Stunden bei 100 °C gerührt. Danach wurde abgekühlt, mit Salzsäure angesäuert, 2 Stunden bei Raumtemperatur gerührt, mit Kaliumhydroxid neutralisiert und der entstandene Salzkuchen abfiltriert. Danach wurden Wasser und Dimethylsulfoxid abdestilliert, der Rückstand mit Toluol versetzt, auf 120 °C erhitzt und filtriert. Nach dem Einengen des Filtrats wurde der Rückstand im Volumenverhältnis 1:1 mit Ethylenglycol versetzt und mit Cyclohexan perforiert.

Es fielen 45 g [2-(2-Ethoxyethoxy)ethoxy]-methyl-18-Krone-6 als ölige Flüssigkeit in einer Ausbeute von 55% an. Reinheit 96%ig.

Beispiel 3

Herstellung von Allyloxymethyl-18-Krone-6

a) Einer Vorlage aus 3,3 g Kaliumhydroxid, gelöst in 375 g Triethylenglycol wurden bei 100 °C innerhalb von 2 Stunden 57,1 g Allylglycidether zudosiert. Danach wurde noch 1 Stunde bei 150 °C gerührt, anschliessend abgekühlt, mit Salzsäure neutralisiert und schliesslich Wasser und überschüssiges Triethylenglycol im Vakuum abdestilliert. Der Rückstand wurde fraktioniert destilliert. Es fielen 108 g Allyloxymethyl-tetraethylenglycol als ölige Flüssigkeit in einer Ausbeute von 80% an.

b) 54,8 g Allyloxymethyl-tetraethylenglycol, 43,0 g 2,2'-Dichlordiethylether, 74,0 g einer 50 Gew.-%igen wässrigen Lösung von Kaliumhydroxid und 80 ml Dimethylsulfoxid wurden 100 Stunden lang bei 12 °C gerührt. Danach wurde auf 80 °C erwärmt und die Reaktionsmischung noch 1 Stunde bei 80 °C gerührt. Nach dem Abkühlen wurde das Reaktionsgemisch mit Salzsäure angesäuert, 1 Stunde bei Raumtemperatur gerührt und mit Kaliumhydroxid neutralisiert. Danach wurde der entstandene Salzkuchen abfiltriert. Vom Filtrat wurden Wasser und Dimethylsulfoxid entfernt, der Rückstand mit Toluol versetzt, auf 120 °C erhitzt und filtriert. Nach Abziehen des Toluols wurde das Reaktionsgemisch im Volumenverhältnis 1:1 mit Ethylenglycol versetzt und mit Cyclohexan perforiert. Der Extrakt wurde eingeengt, erneut im Volumenverhältnis 1:1 mit Ethylenglycol versetzt und die Perforation wiederholt.

Schliesslich wurde Cyclohexan abdestilliert. Es verblieben 37,7 g an Allyloxymethyl-18-Krone-6 als ölige Flüssigkeit in einer Ausbeute von 53%.

Beispiel 4

Herstellung von N-Allyl-aza-18-Krone-6

116 g N-Allyl-diethanolamin, 280 g 1,1'-Oxybis [2-(2-chlorethoxy)ethan], 270 g einer 54 Gew.-%igen wässrigen Lösung von KOH und 320 ml Dimethylsulfoxid wurden 30 Stunden bei 20 °C gerührt. Danach wurde auf 80 °C erwärmt und das Reaktionsgemisch noch 1 Stunde bei dieser Temperatur gerührt. Nach dem Abkühlen des Reaktionsgemisches wurde mit Salzsäure auf pH = 2 gestellt und 1 Stunde bei Raumtemperatur gerührt. Danach wurde das Reaktionsgemisch mit Kalilauge auf pH 13 gebracht. Nach Abdestillieren des Wassers wurde mit Dioxan versetzt und abfiltriert. Vom Filtrat wurden Dioxan und Dimethylsulfoxid abdestilliert, der Rückstand mit 500 ml Toluol versetzt, auf 120 °C erwärmt und filtriert. Nach Abziehen des Toluols wurde im Volumenverhältnis 1:1 mit Ethylenglycol versetzt und mit Cyclohexan perforiert. Es wurde zunächst Cyclohexan abgettrennt, erneut im Volumenverhältnis 1:1 mit Ethylenglycol versetzt und die Perforation wiederholt. Nach Abdestillieren des Extraktionsmittels verblieben 120 g N-Allyl-aza-18-Krone-6 als ölige Flüssigkeit in einer Ausbeute von 48%.

Beispiel 5

N-Methyl-aza-18-Krone-6

23,8 g N-Methyl-diethanolamin, 70,0 g 1,1'-Oxybis[2-(2-Chlorethoxy)ethan], 69,1 g einer 54 Gew.-%igen wässrigen Lösung von KOH und 100 ml Dimethylsulfoxid wurden 24 Stunden bei 20 °C gerührt. Das Reaktionsgemisch wurde anschliessend noch 4 Stunden auf 35 °C und zuletzt 1 Stunde auf 80 °C erwärmt. Nach dem Abkühlen wurde das Reaktionsgemisch mit Salzsäure auf pH 2 gestellt, 1 Stunde bei Raumtemperatur gerührt und schliesslich mit Kalilauge auf pH = 11

gebracht. Danach wurde Wasser abdestilliert und die ausgefallenen Salze durch Filtration abgetrennt. Danach wurde vom Filtrat Dimethylsulfoxid abdestilliert. Schliesslich wurde mit 100 ml Ethylenglycol versetzt und 10 Stunden bei 20 bis 40 °C mit Cyclohexan perforiert. Nach Abdestillieren des Extraktionsmittels verblieben 27,7 g N-Methyl-aza-18-Krone-6 als ölige Flüssigkeit in einer Ausbeute von 49%.

Beispiel 6
Herstellung von Trimethyl-18-Krone-6

40,4 g eines technischen Isomerengemisches von Tripropylenglycol, 57,9 g 1,2-Bis-(2-chlorethoxy)ethan, 69,1 g einer 54 Gew.-%igen wässrigen Lösung von KOH und 80 ml Dimethylsulfoxid wurden 70 Stunden bei 20 °C gerührt. Danach wurde auf 80 °C erwärmt und noch 1 Stunde bei dieser Temperatur gerührt. Nach dem Abkühlen wurde das Reaktionsgemisch mit Salzsäure auf pH 2 gestellt, 1 Stunde bei Raumtemperatur gerührt und anschliessend mit Kalilauge neutralisiert. Danach wurde filtriert und vom Filtrat Wasser und Dimethylsulfoxid abdestilliert. Der Rückstand wurde in 100 ml Toluol aufgenommen, auf 120 °C erwärmt und filtriert. Nach Abdestillieren des Lösungsmittels wurde fraktioniert destilliert. Es fielen bei 0,01 bar im Temperaturbereich von 165 °C bis 192 °C 31,6 g eines Isomerengemisches von Trimethyl-18-Krone-6 als ölige Flüssigkeit an. Die Ausbeute betrug 47%.

**Patentanspruch**

Verfahren zur Herstellung von makrocyclischen Polyethern, die aus gleichen oder verschiedenen Struktureinheiten der Formel

$$-[CR^1R^2-CR^3R^4-A]_x-$$

aufgebaut sind, wobei

A für etherischen Sauerstoff und die Gruppe

$$\begin{matrix} | \\ -N-R^5 \end{matrix}$$

steht,

$R^1$, $R^2$, $R^3$, $R^4$ und $R^5$ Wasserstoff, Alkyl mit 1 bis 18 Kohlenstoffatomen, Alkoxymethyl mit 1 bis 18 Kohlenstoffatomen im Alkoxyrest, Alkenyloxymethyl mit 2 bis 18 Kohlenstoffatomen im Alkenyloxyrest, Alkoxyethyl mit 1 bis 18 Kohlenstoffatomen im Alkoxyrest, Alkenyloxyethyl mit 2 bis 18 Kohlenstoffatomen im Alkenyloxyrest, Alkoxypolyethoxymethyl mit 1 bis 3 Ethylenoxy-Einheiten, Alkoxypolyethoxyethyl mit 1 bis 3 Ethylenoxy-Einheiten, Alkenyl mit 3 bis 18 Kohlenstoffatomen, Phenyl, Phenyloxymethyl, Benzyl und Benzyloxymethyl bedeuten und

x eine ganze Zahl von 5 bis 10 ist, durch

a) Cyclisieren von entsprechende Struktureinheiten aufweisenden α,ω-Diolen mit entsprechende Struktureinheiten aufweisenden α,ω-Dichloriden oder

b) durch Cyclisieren von entsprechende Struktureinheiten aufweisenden α,ω-Chlorhydrinen in Gegenwart von Kaliumhydroxid unter Verwendung von Dimethylsulfoxid als Lösungsmittel, dadurch gekennzeichnet, dass die Cyclisierungsreaktion in Gegenwart von 18 bis 50 Gew.-%, bezogen auf das Gesamtgewicht des Gemisches Wasser/Dimethylsulfoxid/Kaliumhydroxid, an Wasser durchgeführt wird, das zusätzlich zu ggf. bei der Cyclisierungsreaktion gebildetem Wasser dem Reaktionsgemisch zugeführt wurde.

**Revendication**

Procédé pour préparer des polyéthers macrocycliques formés de motifs structurels, identiques ou différents, de formule:

$$-[CR^1R^2-CR^3R^4-A]_x-$$

dans laquelle:

A représente un oxygène de fonction étheroxyde et le groupe:

$$\begin{matrix} | \\ -N-R^5 \end{matrix}$$

$R^1$, $R^2$, $R^3$, $R^4$ et $R^5$ représentent chacun un atome d'hydrogène, un groupe alkyle ayant 1 à 18 atomes de carbone, alcoxyméthyle ayant 1 à 18 atomes de carbone dans le reste alcoxy, alcényloxyméthyle ayant 2 à 18 atomes de carbone dans le reste alcényloxy, alcoxyéthyle ayant 1 à 18 atomes de carbone dans le reste alcoxy, alcényloxyéthyle ayant 2 à 18 atomes de carbone dans le reste alcényloxy, alcoxypolyéthoxyméthyle ayant 1 à 3 motifs éthylène oxy, alcoxypolyéthoxyéthyle ayant 1 à 3 motifs éthylènoxy, alcényle comportant 3 à 18 atomes de carbone, phényle, phényloxyméthyle, benzyle et benzyloxyméthyle, et

x est un nombre entier valant 5 à 10, par

a) cyclisation de diols-α,ω présentant des motifs structurels correspondants avec des dichlorures-α,ω présentant des motifs structurels correspondants, ou

b) par cyclisation de chlorhydrines-α,ω présentant des motifs structurels correspondants,

en présence de l'hydroxyde de potassium et en utilisant du diméthylsulfoxyde comme solvant, procédé caractérisé en ce qu'on conduit la réaction de cyclisation en présence de 18 à 50% en poids d'eau par rapport au poids total du mélange eau/diméthyl-sulfoxyde/hydroxyde de potassium, eau que l'on ajoute au mélange réactionnel en plus de l'eau éventuellement formée dans la réaction de cyclisation.

**Claim**

Process for the preparation of macrocyclic polyethers composed of identical or different structural units of the formula

$$-[CR^1R^2-CR^3R^4-A]_x-$$

in which A represents ether oxygen and the group

$$-\overset{|}{N}-R^5$$

$R^1$, $R^2$, $R^3$, $R^4$ and $R^5$ denote hydrogen, alkyl having 1 to 18 carbon atoms, alkoxymethyl having 1 to 18 carbon atoms in the alkoxy radical, alkenyloxymethyl having 2 to 18 carbon atoms in the alkenyloxy radical, alkoxyethyl having 1 to 18 carbon atoms in the alkoxy radical, alkenyloxyethyl having 2 to 18 carbon atoms in the alkenyloxy radical, alkoxypolyethoxymethyl having 1 to 3 ethyleneoxy units, alkoxypolyethoxyethyl having 1 to 3 ethyleneoxy units, alkenyl having 3 to 18 carbon atoms, phenyl, phenoxymethyl, benzyl and benzyloxymethyl, and

x is an integer from 5 to 10,

a) by cyclizing α,ω-diols containing appropriate structural units by means of α,ω-dichlorides containing appropriate structural units, or

b) by cyclizing α,ω-chlorohydrins containing appropriate structural units,

in the presence of potassium hydroxide and using dimethyl sulphoxide as solvent, characterized in that the cyclization reaction is carried out in the presence of 18 to 50% by weight of water, relative to the total weight of the water/dimethyl sulphoxide/potassium hydroxide mixture, this water having been added to the reaction micture in addition to water which may be formed in the course of the cyclization reaction.